# FASCICULE DE BREVET EUROPEEN

(11) **EP 0 954 252 B1**
(45) Date de publication et mention de la délivrance du brevet: **07.07.2004**
(21) Numéro de dépôt: 97952976.5
(22) Date de dépôt: 22.12.1997
(51) Int. Cl.: A61C 19/045, G09B 23/28

(54) **DISPOSITIF DE DETERMINATION D'UN DEPLACEMENT ENTRE DEUX MOULAGES DENTAIRES AU MOYEN D'UN SCANNER A RAYONS X**
VORRICHTUNG ZUR FESTSTELLUNG EINER VERSCHIEBUNG ZWISCHEN ZWEI ZAHNÄRTZLICHEN ABDRUCKEN MITTELS EINES RONTGENABTASTERS
DEVICE FOR DETERMINING A MOVEMENT BETWEEN TWO DENTAL CAST PROFILES USING AN X-RAY SCANNER

(30) Priorité: 20.12.1996 FR 9616066
(43) Date de publication de la demande: 10.11.1999
(73) Titulaire: UNIVERSITE JOSEPH FOURIER, F-38041 Grenoble Cédex (FR)
(72) Inventeur: BETTEGA, Georges, F-38000 Grenoble (FR); CINQUIN, Philippe, F-38000 Grenoble (FR); LAVALLEE, Stéphane, F-38000 Grenoble (FR); RAPHAEL, Bernard, F-38330 Saint Ismier (FR)
(74) Mandataire: de Beaumont, Michel
(86) Numéro de dépôt international: PCT/FR1997/002383
(87) Numéro de publication internationale: WO 1998/027892

(56) Documents cités:
- EP-A- 0 373 077
- EP-A- 0 375 982
- DE-U- 9 300 356
- US-A- 4 409 616
- US-A- 5 340 309
- KATSUYUKI YAMAMOTO ET AL.: "Measurements of Dental Cast Profile and Three-Dimensional Tooth Movement During Orthodontic Treatment" IEEE TRANSACTIONS ON BIOMEDICAL ENGINEERING, vol. 38, no. 4, avril 1991, NEW YORK, US, pages 360-365, XP000235767

## Description

La présente invention concerne le domaine des appareils utilisés lors d'interventions en chirurgie orthognatique ou en préparation de celles-ci. De telles interventions sont des interventions de chirurgie réparatrice, en particulier, d'un défaut de positionnement des mâchoires l'une par rapport à l'autre. Une intervention en chirurgie orthognatique consiste notamment à pratiquer des ostéotomies du maxillaire et/ou de la mandibule pour les repositionner correctement par rapport au reste du crâne en recréant un articulé dentaire déficient.

La préparation d'une telle intervention chirurgicale requiert la mise en oeuvre de techniques orthodontiques et radiologiques.

On commence généralement par réaliser un moulage mandibulaire et un moulage maxillaire donnant les implantations respectives des dents du patient dans les segments osseux, respectivement mandibulaire et maxillaire. Les moulages, généralement en plâtre, servent à simuler le déplacement relatif qui doit être apporté aux mâchoires pour recréer l'articulé dentaire. Pour permettre au chirurgien de respecter ces positions relatives simulées, on réalise, à partir des moulages dentaires, une plaque comportant, sur chacune de ses faces, des empreintes des dents des deux moulages. Une telle plaque, dite d'intercuspidation, sert à maintenir les moulages ou les mâchoires dans des positions relatives où les dents sont en occlusion.

Comme l'intervention chirurgicale comprend généralement des ostéotomies des deux mâchoires, on réalise, le plus souvent, deux plaques d'intercuspidation à partir des moulages dentaires, en plus d'une plaque d'intercuspidation, dite initiale, reliant les deux mâchoires dans leur position d'occlusion avant intervention.

Une plaque, dite intermédiaire, détermine le déplacement prévisible du maxillaire par rapport à la mandibule alors que celle-ci est dans sa position d'origine (préopératoire). Cette plaque permet au chirurgien de replacer le maxillaire sur le crâne dans la position définitive souhaitée avant d'intervenir sur la mandibule. Une plaque, dite définitive, fixe l'objectif occlusal à atteindre chirurgicalement et sert donc à positionner correctement la mandibule sur le crâne en fixant la position de la mandibule par rapport au maxillaire précédemment replacé.

La préparation de l'acte chirurgical fait également appel à une radiographie de profil du patient permettant, notamment, de procéder à une simulation approximative du geste opératoire.

Cette simulation est réalisée manuellement à partir d'un calque apposé sur la radiographie. Par exemple, on commence par relever les contours de la mandibule. On déplace ensuite le calque pour reproduire approximativement, sur celui-ci, l'occlusion postopératoire souhaitée, puis on trace les contours maxillaires. L'ensemble maxillo-mandibulaire tracé sur le calque est ensuite déplacé en bloc en respectant des normes céphalométriques, les rapports labiaux ainsi que d'autres critères connus pour ce genre d'intervention. On définit ainsi radiologiquement et approximativement le sens et l'amplitude des déplacements des mâchoires. Les résultats de cette simulation sont comparés et ajustés en fonction du déplacement relatif de la mandibule et du maxillaire prévu au moyen des plaques d'intercuspidation.

La simulation actuelle d'une intervention de chirurgie orthognatique s'effectue donc essentiellement manuellement. De plus, cette simulation ne s'effectue que dans deux dimensions à partir d'une vue en plan de profil du crâne.

Le développement des scanners associés à des systèmes de traitement d'images permet d'obtenir des vues en trois dimensions du crâne d'un patient. De tels systèmes seraient particulièrement utiles pour effectuer une simulation en trois dimensions d'une intervention de chirurgie orthognatique. En particulier, on sait isoler les unes des autres différentes parties des images en trois dimensions reconstituées à partir de coupes prises au scanner. Ainsi, on pourrait isoler, du reste du crâne, une partie correspondant au maxillaire et une partie correspondant à la mandibule. Cela permettrait de simuler, au moyen du système de traitement d'images, des déplacements relatifs de ces éléments les uns par rapport aux autres. Toutefois, la seule utilisation de tels systèmes de traitement d'images en trois dimensions pour une simulation d'une intervention de chirurgie orthognatique reste pour l'instant impossible pour plusieurs raisons.

En premier lieu, la précision d'un scanner est incompatible avec le besoin de précision d'un articulé dentaire. En effet, la précision de positionnement des mâchoires requise pour l'articulé dentaire est de l'ordre du dixième de millimètre alors que le pas minimal entre deux tomographies au scanner est de l'ordre de deux à cinq millimètres. On ne peut donc pas reproduire de façon précise, les positions initiales respectives de la mandibule et du maxillaire au moyen du scanner.

En second lieu, les amalgames (plombages) des dents créent des artefacts qui se traduisent par des taches imprécises sur les images issues du scanner. Il n'est donc pas possible de reporter, sur une vue en trois dimensions, la position exacte des dents à partir des images issues du scanner pour obtenir l'articulé dentaire.

On connaît toutefois une technique de préparation et d'assistance d'un geste opératoire en chirurgie orthognatique ayant recours à un scanner. Cette technique consiste à fixer trois vis en titane sur le maxillaire du patient. On réalise ensuite, à partir de coupes prises au scanner du crâne du patient, un modèle en résine du crâne. Les vis apparaissant sur les vues du scanner, elles sont reproduites sur le modèle. Les vis servent à positionner par rapport au crâne une armature métallique de réception d'une plaque d'intercuspidation finale. Cette plaque est réalisée à partir de moulages dentaires maxillaire et mandibulaire pris sur le patient. Une fois les moulages réalisés, on découpe, sur le modèle en résine, le maxillaire pour le remplacer par le moulage correspondant. Le moulage maxillaire est fixé au modèle dans la position définitive souhaitée. Puis on découpe la mandibule du modèle pour la remplacer par un moulage réalisé préalablement sur le patient.

La position du moulage mandibulaire est donnée, par rapport au maxillaire, par la plaque d'intercuspidation qui est alors couplée rigidement à l'armature métallique constituant un système de transfert de la position de la plaque entre le modèle et le patient. L'armature est alors ramenée sur le patient dans la position définie par les trois vis du maxillaire et est fixée au crâne du patient par deux vis supplémentaires. La position du système de transfert étant désormais figée par ces deux vis, on procède à l'ostéotomie du maxillaire que l'on repositionne correctement à l'aide de la plaque d'intercuspidation qui est couplée rigidement au système de transfert. Enfin, on réalise l'ostéotomie de la mandibule et on la positionne correctement à l'aide de la plaque d'intercuspidation.

Une telle technique présente plusieurs inconvénients. D'une part, elle nécessite une intervention chirurgicale supplémentaire pour mettre en place les vis dans la bouche du patient. D'autre part, elle nécessite la réalisation d'un modèle du crâne en résine, ce qui est particulièrement coûteux. En outre, la simulation est effectuée, de façon empirique, au moyen du modèle en résine et ne permet pas de prendre en compte les normes céphalométriques à partir de données précises.

La présente invention vise à proposer un nouveau système de simulation en trois dimensions pour des interventions en chirurgie orthognatique.

Pour atteindre cet objet, la présente invention prévoit une plaque d'intercuspidation de définition d'une occlusion entre deux moulages dentaires, respectivement mandibulaire et maxillaire, comportant, en saillie de son extrémité frontale, un repère visible au moyen d'un scanner à rayon X et propre à définir un premier référentiel dans des images en trois dimensions reconstituées à partir de vues issues du scanner.

Selon un mode de réalisation de la présente invention, ledit repère comporte au moins deux bords rectilignes non parallèles entre eux.

Selon un mode de réalisation de la présente invention, ledit repère est constitué d'au moins deux tiges reliées à la plaque d'intercuspidation et formant entre elles un angle déterminé.

Selon un mode de réalisation de la présente invention, lesdites tiges sont creuses et ouvertes à leur extrémité distale de la plaque d'intercuspidation.

La présente invention concerne également un dispositif de détermination d'un déplacement en trois dimensions entre des première et deuxième positions relatives de deux moulages dentaires, comportant un premier module, associé à un premier moulage dentaire et localisable en position et en orientation par un dispositif de localisation, un deuxième module, associé à un second moulage dentaire et localisable par le dispositif de localisation, une première plaque d'intercuspidation définissant la première position relative entre les deux moulages, la première plaque étant localisable par le dispositif de localisation, des moyens de mémorisation de la position du deuxième module par rapport au premier module quand la position relative des moulages est fixée par la première plaque d'intercuspidation, des moyens de définition de la deuxième position relative des moulages, et des moyens de calculs propres à déterminer le déplacement en trois dimensions du deuxième module entre les deux positions.

Selon un mode de réalisation de la présente invention, les moyens de définition de la deuxième position relative des moulages sont constitués d'une deuxième plaque d'intercuspidation, le dispositif comportant en outre des moyens de mémorisation de la position relative du deuxième module par rapport au premier module quand la position des moulages est fixée par la deuxième plaque d'intercuspidation, et des moyens de calculs propres à déterminer le déplacement en trois dimensions du deuxième module entre les deux positions.

Selon un mode de réalisation de la présente invention, les moyens de définition de la deuxième position relative des moulages sont constitués d'un dispositif de numérisation des surfaces des moulages et de moyens de traitement d'images.

Selon un mode de réalisation de la présente invention, le dispositif de localisation est constitué d'un localisateur optique, les modules localisables comportant des diodes d'émission d'un rayonnement infrarouge.

Selon un mode de réalisation de la présente invention, le dispositif de localisation est constitué de plusieurs caméras vidéo associées à des moyens de traitement d'images, lesdits modules localisables étant constitués de motifs graphiques.

La présente invention concerne en outre un système de simulation d'un acte de chirurgie orthognatique, comportant un scanner à rayons X, un dispositif de localisation en trois dimensions de positions relatives de moulages dentaires associés chacun à un module localisable en position et en orientation par le dispositif de localisation, et un système de traitement d'images propre à combiner des images tomographiques issues du scanner avec des données de positionnement issues du dispositif de localisation pour obtenir des images en trois dimensions.

Selon un mode de réalisation de la présente invention, le dispositif de localisation fait partie d'un dispositif de détermination d'un déplacement en trois dimensions entre des première et deuxième positions relatives des deux moulages dentaires.

Selon un mode de réalisation de la présente invention, une première position est fixée par une première plaque d'intercuspidation.

Selon un mode de réalisation de la présente invention, une relation entre les premier et deuxième référentiels est déterminée à partir d'un module additionnel d'un élément de référence propre à être associé mécaniquement au repère de la première plaque d'intercuspidation.

Selon un mode de réalisation de la présente invention, le repère de la première plaque d'intercuspidation est couplé rigidement à un troisième module localisable qui définit un deuxième référentiel associé à la première plaque.

Ces objets, caractéristiques et avantages, ainsi que d'autres de la présente invention seront exposés en détail dans la description suivante de modes de réalisation et de mise en oeuvre particuliers faite à titre non-limitatif en relation avec les figures jointes parmi lesquelles :
La figure 1 représente un mode de réalisation d'une plaque d'intercuspidation selon la présente invention ;
les figures 2 et 3 illustrent schématiquement un premier mode de mise en oeuvre d'un dispositif de détermination d'un déplacement en trois dimensions entre deux moulages dentaires selon la présente invention ; et
la figure 4 représente un mode de réalisation d'un élément de référence selon l'invention associé à un repère visible au scanner d'une plaque d'intercuspidation telle que représentée à la figure 1.

Une caractéristique de la présente invention est d'associer, à un système de traitement d'images fournissant des images en trois dimensions à partir d'un scanner à rayon X, un dispositif de détermination d'un déplacement en trois dimensions entre des première et deuxième positions relatives de deux moulages dentaires. Les images issues du scanner et les positions et orientations des moulages déterminées par le dispositif sont combinées dans le système de traitement d'images pour obtenir un système précis de simulation en trois dimensions.

L'invention sera décrite par la suite à partir d'un exemple de cas pratique mettant en oeuvre le système de simulation selon l'invention. On notera toutefois que la succession d'étapes indiquée ci-dessous pourra être modifiée.

On commence par réaliser deux moulages dentaires, respectivement maxillaire et mandibulaire, reproduisant les positions respectives des dents dans chacune des mâchoires.

On réalise également une première plaque d'intercuspidation, dite initiale, correspondant à la position mandibulaire en occlusion habituelle sur le maxillaire. Cette plaque est généralement réalisée par une prise d'empreinte en bouche du patient.

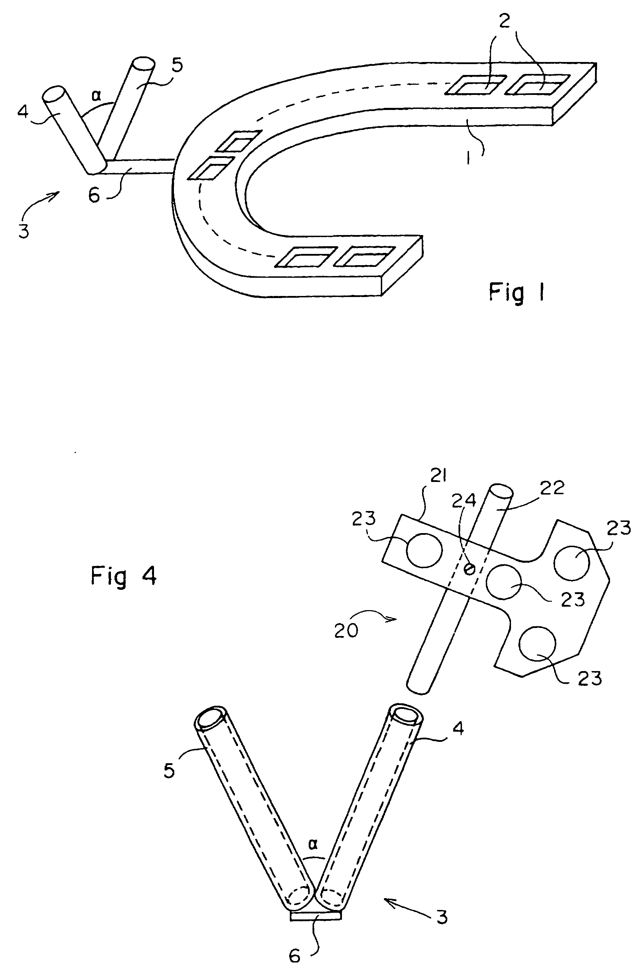

La figure 1 représente un mode de réalisation d'une plaque d'intercuspidation initiale selon la présente invention.

Une telle plaque d'intercuspidation 1 est réalisée par des moyens classiques. La plaque 1 comporte, sur chacune de ses faces, des empreintes 2 des dents des deux mâchoires. Les empreintes 2 sont non-traversantes mais suffisamment profondes pour que les dents reconstituées sur les moulages soient en position d'occlusion lorsque la plaque 1 est insérée entre les deux moulages dentaires. A la figure 1, seules quelques empreintes 2 ont été représentées.

Selon la présente invention, la plaque d'intercuspidation 1 porte un repère physique 3. Le rôle du repère 3 est de définir un référentiel en trois dimensions, dans des images reconstituées à partir de coupes prises au scanner (non représenté), qui soit localisable, directement ou indirectement, par un dispositif externe (non représenté à la figure 1), appelé localisateur, de détermination des positions et orientations relatives des moulages pour combiner, au sein du système de traitement d'images, les images issues du scanner avec des données de position et d'orientation fournies par le localisateur. Le localisateur peut être un système de stéréovision, un capteur optique de positions de diodes, un système à ultrasons, un système de capteurs électromagnétiques ou autres.

De préférence, le repère 3 est saillant de l'extrémité avant, ou frontale, de la plaque 1 de manière à être accessible lorsque la plaque 1 est associée au moulage dentaire.

Dans l'exemple représenté à la figure 1, le repère 3 est constitué de deux tiges 4 et 5 comprises dans un plan approximativement perpendiculaire au plan de la plaque 1. Les tiges 4 et 5 sont portées, par une de leurs extrémités respectives, par un support 6. Le support 6 est constitué, par exemple, d'une tige approximativement coplanaire à la plaque 1, faisant saillie à l'avant de la plaque 1. Les tiges 4 et 5 définissent un angle α entre elles en partant du support 6.

A titre de variante non représentée, les tiges 4 et 5 peuvent être portées, par une de leurs extrémités respectives, directement par la plaque 1. Dans ce cas, le plan contenant les tiges 4 et 5 est incliné vers l'avant par rapport au plan perpendiculaire au plan de la plaque 1, pour éviter le nez du patient lorsque cette plaque 1 est placée entre les dents du patient. Bien entendu, cette variante peut être combinée avec l'emploi d'un support 6 saillant de la plaque 1, l'angle d'inclinaison du plan contenant les tiges 4 et 5 étant alors adapté au débord introduit par le support 6.

Une caractéristique d'un repère 3 selon la présente invention est qu'il est constitué en un matériau ne générant pas d'artefact lors d'une visualisation au scanner. Par exemple, les tiges 4 et 5 et le support 6 éventuel sont en aluminium ou en plastique rigide visible au scanner à rayons X.

Selon un autre mode de réalisation non représenté, le repère 3 est constitué d'une forme géométrique quelconque comportant au moins deux bords, rectilignes et non parallèles, visibles au scanner. Il pourra s'agir, par exemple, d'une plaque en forme d'étoile portée par un support en saillie de la partie frontale de la plaque 1.

Une autre caractéristique du repère 3 selon l'invention est qu'il sert également à localiser la plaque 1 au moyen d'un dispositif de localisation, ou localisateur, en trois dimensions. Pour ce faire, le repère 3 constitue, de lui-même, un repère visible par un tel localisateur ou est destiné à être couplé rigidement à un repère visible par le localisateur comme cela sera décrit par la suite.

Une fois la plaque d'intercuspidation initiale réalisée, on effectue au moyen du scanner une série de coupes tomographiques du crâne avec cette plaque en position entre les mâchoires. On obtient alors, au moyen du système de traitement d'images et de manière classique, une modélisation en trois dimensions du crâne et des mâchoires.

Comme les tiges 4 et 5 ne sont pas parallèles entre elles, un traitement de recalage entre les points des tiges 4 et 5 présents dans plusieurs images qui correspondent à des coupes parallèles entre elles et un modèle géométrique connu de ces tiges permet de connaître la position et l'orientation d'un référentiel défini par les tiges et associé à la plaque 1, c'est-à-dire du repère 3, dans le référentiel des images fournies par le système de traitement.

On notera que le repère 3 peut comporter un nombre plus important de tiges, par exemple trois tiges non-coplanaires et non-parallèles deux à deux, pour définir un référentiel de façon plus fiable et plus précise.

Pour permettre une simulation de l'intervention chirurgicale à partir des images en trois dimensions reconstituées, on doit transférer, dans le système de traitement d'images, le déplacement relatif en trois dimensions qui doit être apporté aux mâchoires, respectivement maxillaire et mandibulaire.

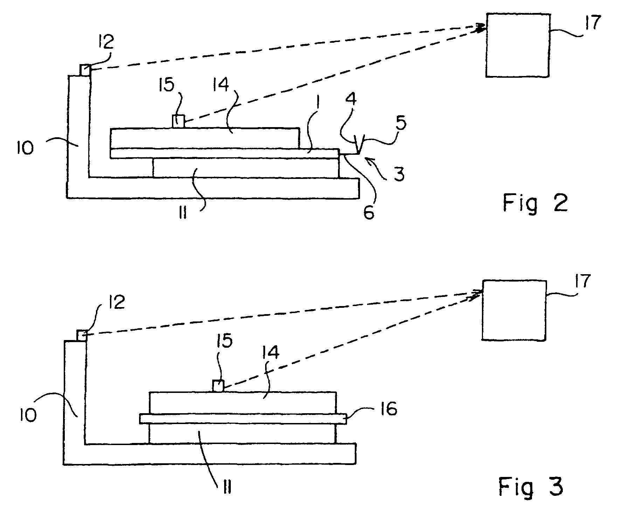

Pour ce faire, on utilise un dispositif de détermination de déplacement dont un premier mode de mise en oeuvre est décrit ci-dessous en relation avec les figures 2 et 3.

Selon ce premier mode de mise en oeuvre de l'invention, on réalise, à partir des moulages dentaires, une deuxième plaque d'intercuspidation, dite finale, correspondant à l'objectif occlusal souhaité.

Cette plaque d'intercuspidation finale peut être réalisée, de façon classique, en plaçant les deux moulages dentaires sur un articulateur réglable pour mettre en coïncidence, selon l'objectif postopératoire souhaité, certains repères anatomiques dentaires pris sur le moulage mandibulaire avec d'autres repères pris sur le moulage maxillaire.

La plaque d'intercuspidation finale peut également être réalisée, selon l'invention, à partir d'une numérisation des surfaces respectives des moulages en désignant des points de repères sur ces surfaces dans un système informatique. On pourra alors utiliser un articulateur motorisé pour placer les deux moulages dans la position finale souhaitée et réaliser alors la plaque finale de façon plus précise. La numérisation de surfaces dentaires est une technique connue.

Selon le mode de mise en oeuvre décrit, on utilise un localisateur optique permettant de définir, avec précision, les positions et orientations respectives des moulages mandibulaire et maxillaire. Un tel localisateur optique est connu de l'art antérieur. Il utilise généralement des modules, équipés de diodes d'émissions infrarouges, couplés rigidement aux éléments à localiser. Les positions et orientations respectives des modules sont détectées au moyen d'un appareil 17 pourvu de trois barrettes (non représentées) d'éléments photosensibles à transfert de charge (CCD) et de lentilles cylindriques qui projettent l'image des diodes à infrarouges sur ces barrettes. La disposition des barrettes, une horizontale et deux verticales, permet de définir la position de n'importe quel point présent dans le champ du détecteur 17 avec une bonne précision, généralement de 0,1 mm dans le plan frontal du détecteur et de 0,15 mm en profondeur. L'association des positions respectives des diodes d'un même module (généralement deux paires de diodes dans des directions perpendiculaires) permet de déterminer la position et l'orientation de l'élément associé au module.

Selon la présente invention, le dispositif de détermination du déplacement en trois dimensions entre les première et deuxième positions relatives des deux moulages dentaires, respectivement fixées par les plaques d'intercuspidation initiale et finale, comporte un support 10 de réception du moulage mandibulaire 11, la position du moulage 11 étant fixe par rapport au support 10. Le support 10 ou le moulage 11 est associé à un premier module 12 de diodes à infrarouges. Ce premier module 12 définit un référentiel en trois dimensions, associé au moulage mandibulaire 11 et fixe par rapport au détecteur 17.

Lors d'une première étape (figure 2), on place la plaque d'intercuspidation initiale 1 et le moulage maxillaire 14, en relation d'occlusion sur le moulage 11. Le moulage maxillaire 14 est pourvu d'un module 15 de localisation définissant un référentiel en trois dimensions associé au moulage maxillaire.

On effectue une localisation des modules 12 et 15 par le localisateur optique. Cette localisation donne la position et l'orientation des modules dans un référentiel associé au détecteur 17, donc au localisateur. On détermine alors, par des moyens de calculs, les positions relatives des deux référentiels respectivement associés au moulage mandibulaire et au moulage maxillaire dans la position d'occlusion habituelle. On peut ainsi déterminer la relation (matrice de passage) entre le référentiel associé au moulage mandibulaire et le référentiel associé au moulage maxillaire dans sa position initiale ainsi que les relations respectives de ces deux référentiels avec le référentiel associé au détecteur 17.

Dans une deuxième étape illustrée par la figure 3, on remplace la plaque d'intercuspidation initiale 1 par la plaque d'intercuspidation finale réalisée à partir des moulages dentaires. La plaque 16 d'intercuspidation finale définit la position relative des moulages mandibulaire et maxillaire dans l'objectif occlusal souhaité. En effectuant une deuxième série de mesures par le localisateur optique, on obtient les positions et orientations respectives des modules 12 et 15, donc des moulages mandibulaire et maxillaire, dans la position finale souhaitée. On peut alors déterminer la relation entre le nouveau référentiel associé au moulage maxillaire 14 et le référentiel associé au moulage mandibulaire 11 qui n'a pas changé.

Enfin, on détermine à partir des relations respectives entre le référentiel du moulage 11 et les référentiels initial et final du moulage 14, la relation (matrice de passage) entre les deux référentiels, respectivement initial et final, du moulage maxillaire. On connaît ainsi le déplacement en trois dimensions entre les positions, respectivement initiale et finale, du moulage maxillaire 14 par rapport au moulage mandibulaire 11.

Pour permettre l'association des informations de position avec les coupes réalisées au scanner, on détermine la relation entre le référentiel associé au repère 3 et le référentiel associé au détecteur 17.

Cette détermination de la matrice de passage entre ces deux référentiels est, selon le mode de réalisation décrit, effectuée dans une étape supplémentaire au moyen d'un élément de référence dont la figure 4 représente un mode de réalisation destiné à un repère 3 tel que décrit précédemment.

L'élément de référence 20 est constitué d'un module additionnel 21 de diodes à infrarouges 23, couplé rigidement, par exemple par une ou plusieurs vis 24, à un élément rectiligne 22 de liaison avec une tige 4 ou 5 du repère 3. Par exemple, chaque tige 4 ou 5 est creuse et ouverte à son extrémité libre et l'élément de liaison est constitué d'une tige 22 destinée à s'engager dans une des tiges 4 ou 5. La tige 22 possède, quand elle est engagée dans une tige 4 ou 5, deux degrés de liberté (coulissement et rotation) par rapport à celle-ci. Le module 21 définit un référentiel en trois dimensions associé à la tige 22.

On commence par déterminer, au moyen du détecteur 17, les coordonnées de deux points choisis arbitrairement le long de la tige 22 dans son référentiel. On engage alors la tige 22 dans une des tiges (par exemple, 4) du repère 3 et on détermine, à partir d'une mesure de la position du module 21 par rapport au module 12 qui définit un référentiel fixe par rapport au localisateur, les positions respectives des deux points dans le référentiel associé au détecteur 17. On réitère l'opération en engageant la tige 22 dans la tige 5. Ces deux séries de mesures permettent de déterminer, dans le référentiel défini par le module 12, donc dans le référentiel associé au localisateur, les directions respectives des tiges 4 et 5. En recalant mathématiquement ces positions mesurées avec un modèle géométrique des tiges ou directement avec les points des tiges déterminés sur les coupes issues du scanner, on peut alors déterminer la matrice de passage entre les référentiels définis par le repère 3 dans les images en trois dimensions reconstituées à partir des coupes issues du scanner et le référentiel du localisateur.

A titre de variante, l'étape supplémentaire consiste à engager la tige 22 dans une tige (par exemple, 4) du repère 3 et à effectuer plusieurs mesures d'orientation de la tige 22 en la faisant tourner dans la tige 4. On réitère cette opération en faisant tourner la tige 22 dans la tige 5. Puis, on recherche l'axe invariant à toutes les mesures effectuées pour chacune des tiges 4 et 5 dans le référentiel défini par le module 12. Cet axe invariant correspond à la direction de la tige 4, respectivement 5, dans ce référentiel et l'intersection des deux axes obtenus donne l'origine du référentiel associé au repère 3. On en déduit la matrice de passage entre le référentiel associé au repère 3 et le référentiel du localisateur.

En connaissant les différentes relations entre les référentiels, on peut désormais simuler le déplacement à apporter aux positions relatives des mâchoires dans le système de traitement d'images en trois dimensions. Après avoir simulé les ostéotomies respectives des segments osseux de la mandibule et du maxillaire par rapport au reste du crâne en isolant dans les images en trois dimensions issues du scanner une partie correspondant au maxillaire et une partie correspondant à la mandibule, on peut simuler, au moyen du système de traitement d'images, la position finale à donner à la mandibule et au maxillaire indépendamment de la plaque d'intercuspidation initiale. En effet, les positions respectives de la mandibule et du maxillaire sont parfaitement déterminées dans le système de traitement d'image. De fait, l'application, dans les images issues du scanner, de la relation entre les référentiels initial et final du maxillaire déterminés par le localisateur optique, permet d'obtenir un bloc maxillo-mandibulaire corrigé, c'est à dire dans la position correspondant à celle définie par la plaque d'intercuspidation finale.

A titre de variante non représentée, le dispositif de localisation optique peut être remplacé par un dispositif de localisation vidéo utilisant plusieurs caméras (stéréovision) pour localiser les modules respectivement associés au moulage mandibulaire 11, au moulage maxillaire 14 et à la plaque d'intercuspidation 1. Dans ce cas, les modules de définition des trois référentiels sont, par exemple, constitués de motifs graphiques localisables en position et en orientation par une analyse des images issues des caméras vidéo. Le repère physique 3 peut ici définir directement un référentiel lié au dispositif de localisation. On dispose alors d'un même référentiel, côté scanner et côté localisateur. Le même principe s'applique également à un localisateur par émetteurs à ultrasons ou par capteurs électromagnétiques.

Un avantage de la présente invention est que le praticien dispose désormais d'un outil précis de simulation en trois dimensions de l'intervention chirurgicale à réaliser sans qu'il soit nécessaire de lier rigidement des éléments au crâne du patient. En particulier, le déplacement relatif entre le maxillaire et la mandibule peut être pris en compte dans une simulation et dans une analyse céphalométrique complète utilisant les images en trois dimensions issues du scanner.

Un autre avantage de la présente invention est que la connaissance exacte des positions et orientations respectives des différents segments osseux permet de prévoir, au sein du bloc opératoire, des moyens robotisés d'assistance au geste opératoire, la mandibule et le maxillaire pouvant être positionnés très exactement au moyen d'un robot, ou au moyen d'un système de guidage passif, par rapport au crâne du patient dans la position finale souhaitée.

Les positions sont alors données, par exemple, à l'aide d'un localisateur optique fixe dans le bloc opératoire et de la plaque d'intercuspidation 1 mise en bouche du patient juste avant l'opération. Une nouvelle étape de détermination de la position de la plaque 1 permet de transférer toutes les données de la simulation dans le nouveau référentiel associé au localisateur optique. Cette étape peut être effectuée comme précédemment au moyen de l'élément de référence 20. A titre de variante, on pourra également associer le repère 3 à un troisième module de diodes (non représenté) couplé rigidement aux tiges 4 et 5. Ce module définit alors un référentiel en trois dimensions associé à la plaque 1 dans le dispositif de localisation.

On notera que, dans le cas d'un positionnement manuel des différents segments osseux, on réalise toujours une plaque d'intercuspidation qui fixe le déplacement du maxillaire par rapport à la mandibule dans sa position d'origine. Dans le cas d'un positionnement robotisé des segments osseux, le recours à une telle plaque d'intercuspidation intermédiaire n'est plus nécessaire et les deux mâchoires peuvent être repositionnées en même temps.

On notera également que l'invention peut également être mise en oeuvre dans le cas où le maxillaire doit être divisé en plusieurs fragments pour obtenir un positionnement dentaire correct. Dans ce cas, on utilise plusieurs modules 15 respectivement associés à chaque fragment et on détermine, pour chaque ensemble ainsi constitué (fragment de moulage et module de diodes), une matrice de déplacement dans le référentiel du localisateur puis dans le référentiel du scanner.

Selon un deuxième mode de mise en oeuvre de la présente invention, le localisateur est utilisé pour mettre en oeuvre la première étape décrite en relation avec la figure 2 ainsi que l'étape supplémentaire (figure 4) de détermination de la matrice de passage entre le référentiel associé au repère 3 et le référentiel du localisateur. Par contre, la position relative entre les deux moulages dentaires dans la position postopératoire souhaitée est déterminée directement à l'aide du système de traitement d'images, sans recours à une deuxième plaque d'intercuspidation (16, figure 3). Pour ce faire, on numérise les surfaces des deux moulages au moyen d'un capteur de surface, pour obtenir des empreintes numériques dans le système de traitement d'images. Les positions définitives souhaitées entre les mâchoires sont alors directement déterminées dans le système de traitement d'images dans lequel les positions initiales sont connues de façon précise grâce à l'emploi du localisateur et de la première plaque 1 associée au repère 3.

Un avantage de ce mode de mise en oeuvre est qu'il permet d'utiliser un système informatique pour optimiser les critères de positionnement et respecter des contraintes de positionnement de façon fiable et semi-automatique. Un autre avantage est que tout le traitement relatif à la simulation et à la détermination de la position définitive des mâchoires peut ici être réalisé par le chirurgien alors que la plaque d'intercuspidation finale est généralement réalisée par un prothésiste à partir de données fournies par le chirurgien.

Bien entendu, la présente invention est susceptible de diverses variantes et modifications qui apparaîtront à l'homme de l'art. En particulier, la réalisation des outils informatiques et des programmes de calcul des différentes relations entre les référentiels est à la portée de l'homme de l'art en fonction des indications fonctionnelles données ci-dessus.

## Revendications

1. Plaque d'intercuspidation (1) de définition d'une occlusion entre deux moulages dentaires, respectivement mandibulaire (11) et maxillaire (14), **caractérisée en ce qu'**elle comporte, en saillie de son extrémité frontale, un repère (3) visible au moyen d'un scanner à rayon X et propre à définir un premier référentiel dans des images en trois dimensions reconstituées à partir de vues issues du scanner, ledit repère comportant au moins deux bords rectilignes non parallèles entre eux et étant constitué d'au moins deux tiges (4, 5) reliées à la plaque d'intercuspidation et formant entre elles un angle déterminé (α), lesdites tiges étant creuses et ouvertes à leur extrémité distale de la plaque d'intercuspidation.

2. Dispositif de détermination d'un déplacement en trois dimensions entre des première et deuxième positions relatives de deux moulages dentaires, **caractérisé en ce qu'**il comporte :
un premier module (12), associé à un premier moulage dentaire (11) et localisable en position et en orientation par un dispositif de localisation (17) ;
un deuxième module (15), associé à un second moulage dentaire (14) et localisable par le dispositif de localisation ;
une première plaque d'intercuspidation (1) conforme à la revendication 1 et définissant la première position relative entre les deux moulages (11, 14), la première plaque (1) étant localisable par le dispositif de localisation (17) ;
des moyens de mémorisation de la position du deuxième module (15) par rapport au premier module (12) quand la position relative des moulages (11, 14) est fixée par la première plaque d'intercuspidation (1);
des moyens de définition (16) de la deuxième position relative des moulages (11, 14) ; et
des moyens de calculs propres à déterminer le déplacement en trois dimensions du deuxième module (15) entre les deux positions.

3. Dispositif de détermination d'un déplacement en trois dimensions selon la revendication 2, **caractérisé en ce que** les moyens de définition de la deuxième position relative des moulages (11, 14) sont constitués d'une deuxième plaque d'intercuspidation (16), le dispositif comportant en outre :
des moyens de mémorisation de la position relative du deuxième module (15) par rapport au premier module (12) quand la position des moulages (11, 14) est fixée par la deuxième plaque d'intercuspidation (16) ; et
des moyens de calculs propres à déterminer le déplacement en trois dimensions du deuxième module (15) entre les deux positions.

4. Dispositif de détermination d'un déplacement en trois dimensions selon la revendication 2, **caractérisé en ce que** les moyens de définition de la deuxième position relative des moulages (11, 14) sont constitués d'un dispositif de numérisation des surfaces des moulages et de moyens de traitement d'images.

5. Dispositif de détermination d'un déplacement en trois dimensions selon l'une quelconque des revendications 2 à 4, **caractérisé en ce que** le dispositif de localisation est constitué d'un localisateur optique (17), les modules (12, 15, 21) localisables comportant des diodes (23) d'émission d'un rayonnement infrarouge.

6. Dispositif de détermination d'un déplacement en trois dimensions selon l'une quelconque des revendications 2 à 4, **caractérisé en ce que** le dispositif de localisation (17) est constitué de plusieurs caméras vidéo associées à des moyens de traitement d'images, lesdits modules localisables étant constitués de motifs graphiques.

7. Système de simulation d'un acte de chirurgie orthognatique, **caractérisé en ce qu'**il comporte :
un scanner à rayons X ;
un dispositif de détermination d'un déplacement en trois dimensions entre des première et deuxième positions relatives des deux moulages dentaires (11, 14) selon l'une quelconque des revendications 2 à 6 ; et
un système de traitement d'images propre à combiner des images tomographiques issues du scanner avec des données de positionnement issues du dispositif de localisation (17) pour obtenir des images en trois dimensions.

8. Système de simulation selon la revendication 7, **caractérisé en ce qu'**une relation entre les premier et deuxième référentiels est déterminée à partir d'un module additionnel (21) d'un élément de référence (20) propre à être associé mécaniquement au repère (3) de la première plaque d'intercuspidation (1).

9. Système de simulation selon la revendication 7 ou 8, **caractérisé en ce que** le repère (3) de la première plaque d'intercuspidation (1) est couplé rigidement à un troisième module localisable qui définit un deuxième référentiel associé à la première plaque (1).

## Patentansprüche

1. Intercuspidationsplatte (1) zum Bestimmen einer Okklusion zwischen zwei dentalen Abdrücken, nämlich des Unterkiefers (11) und des Oberkiefers (14), **dadurch gekennzeichnet, dass** sie als Vorsprung ihres vorderen Endes eine Kennmarke (3) aufweist, die mit Hilfe eines Röntgenscanners sichtbar und geeignet ist, ein erstes Bezugssystem in dreidimensionalen Bildern zu definieren, die ausgehend von den vom Scanner ausgegebenen Ansichten rekonstruiert wurden, wobei die Kennmarke zumindest zwei gradlinige und nicht zueinander parallele Kanten aufweist und aus mindestens zwei Stangen (4, 5) gebildet ist, die mit der Intercuspidationsplatte verbunden sind und zwischen sich einen bestimmten Winkel (α) einschließen, wobei diese Stangen hohl und an ihrem zu der Intercuspidationsplatte distalen Ende offen sind.

2. Vorrichtung zum Feststellen einer Verschiebung in drei Dimensionen zwischen ersten und zweiten Relativpositionen zweier dentaler Abdrücke, **dadurch gekennzeichnet, dass** sie aufweist:
ein erstes Modul (12), das einem ersten dentalen Abdruck (11) zugeordnet und in seiner Lage und Ausrichtung durch eine Lokalisationseinrichtung (17) lokalisierbar ist;
ein zweites Modul (15), das einem zweiten dentalen Abdruck (14) zugeordnet und durch die Lokalisationseinrichtung lokalisierbar ist;
eine erste Intercuspidationsplatte (1) nach Anspruch 1, die die erste Relativposition zwischen den beiden Abdrücken (11, 14) definiert, wobei die erste Platte (1) durch die Lokalisationseinrichtung (17) lokalisierbar ist;
Speichereinrichtungen für die Position des zweiten Modules (15) in Bezug zu dem ersten Modul (12), wenn die Relativposition der Abdrücke (11, 14) durch die erste Intercuspidationsplatte (1) festgelegt ist;
Einrichtungen (16) zum Feststellen der zweiten Relativposition der Abdrücke (11, 14); und
Recheneinrichtungen, die geeignet sind, die Verschiebung in drei Dimensionen des zweiten Modules (15) zwischen den beiden Positionen zu bestimmen.

3. Vorrichtung zum Feststellen einer Verschiebung in drei Dimensionen nach Anspruch 2, **dadurch gekennzeichnet, dass** die Einrichtungen zum Feststellen der zweiten Relativposition der Abdrücke (11, 14) durch eine zweite Intercuspidationsplatte (16) gebildet sind, wobei die Vorrichtung darüber hinaus aufweist:
Speichereinrichtungen für die Relativposition des zweiten Modules (15) in Bezug zu dem ersten Modul (12), wenn die Position der Abdrücke (11, 14) durch die zweite Intercuspidationsplatte (16) festgelegt ist; und
Recheneinrichtungen, die geeignet sind, die Verschiebung in drei Dimensionen des zweiten Modules (15) zwischen den beiden Positionen zu bestimmen.

4. Vorrichtung zum Feststellen einer Verschiebung in drei Dimensionen nach Anspruch 2, **dadurch gekennzeichnet, dass** die Einrichtungen zum Feststellen der zweiten Relativposition der Abdrücke (11, 14) eine Einrichtung zum Digitalisieren der Oberflächen der Abdrücke und Einrichtungen zur Bildbearbeitung aufweisen.

5. Vorrichtung zum Feststellen einer Verschiebung in drei Dimensionen nach einem der Ansprüche 2 bis 4, **dadurch gekennzeichnet, dass** die Lokalisationseinrichtung aus ein optischer Lokalisierer (17) ist, wobei die lokalisierbaren Module (12, 15, 21) Dioden (23) zur Emission von infraroter Strahlung aufweisen.

6. Vorrichtung zum Feststellen einer Verschiebung in drei Dimensionen nach der einem der Ansprüche 2 bis 4, **dadurch gekennzeichnet, dass** die Lokalisiationseinrichtung (17) mehrere Videokameras aufweist, die den Einrichtungen zur Bildbearbeitung zugeordnet sind, wobei die lokalisierbaren Module mit grafischen Motiven versehen sind.

7. System zur Simulation einer kieferchirurgischen Behandlung, **dadurch gekennzeichnet, dass** es aufweist:
einen Röntgenscanner;
eine Einrichtung zum Feststellen einer Verschiebung in drei Dimensionen zwischen ersten und zweiten Relativpositionen von zwei dentalen Abdrücken (11, 14) nach einem der Ansprüche 2 bis 6; und
ein System zur Bildbearbeitung, das geeignet ist, tomografische Bilder, die vom Scanner ausgegeben wurden, mit Positionsdaten zu kombinieren, die von der Lokalisationseinrichtung (17) ausgegeben wurden, um Bilder in drei Dimensionen zu erhalten.

8. System zur Simulation nach Anspruch 7, **dadurch gekennzeichnet, dass** zwischen dem ersten und zweiten Bezugssystem eine Beziehung ausgehend von einem zusätzlichen Modul (21) eines Referenzelementes (20) bestimmt wird, das mechanisch der Kennmarke (3) der ersten Intercuspidationsplatte (1) zuordenbar ist.

9. System zur Simulation nach Anspruch 7 oder 8, **dadurch gekennzeichnet, dass** die Kennmarke (3) der ersten Intercuspidationsplatte (1) fest mit einem dritten lokalisierbaren Modul gekoppelt ist, das ein zweites Referenzsystem definiert, welches der ersten Platte (1) zugeordnet ist.

## Claims

1. An interscupidation plate (1) for defining an occlusion between two, respectively mandibular (11) and maxillary (14), dental castings, **characterized in that** it includes, protruding from its front end, a guide mark (3) visible by means of an X-ray scanner and adapted to defining a first referential in three-dimensional images reconstructed from scanner views, said guide mark having at least two non-parallel rectilinear edges and being formed of at least two rods (4, 5) connected to the interscupidation plate and forming together a determined angle (α), said rods being hollow and open at their distal end from the interscupidation plate.

2. A device for determining a three-dimensional displacement between first and second relative positions of two dental castings, **characterized in that** it includes:
a first unit (12), associated with a first dental casting (11) and locatable in position and orientation by a localization device (17);
a second unit (15), associated with a second dental casting (14) and locatable by the localization device;
a first interscupidation plate (1) according to claim 1 and defining the first relative position between the two castings (11, 14), the first plate (1) being locatable by the localization device (17);
means for storing the position of the second unit (15) with respect to the first unit (12) when the relative position of the castings (11, 14) is determined by the first interscupidation plate (1);
means for defining (16) the second relative position of the castings (11, 14); and
calculation means adapted to determining the three-dimensional displacement of the second unit (15) between the two positions.

3. The device for determining a three-dimensional displacement of claim 2, **characterized in that** the means for defining the second relative position of the castings (11, 14) are formed of a second interscupidation plate (16), and further including:
means for storing of the relative position of the second unit (15) with respect to the first unit (12) when the position of the castings (11, 14) is determined by the second interscupidation plate (16); and
calculation means adapted to determining the three-dimensional displacement of the second unit (15) between the two positions.

4. The device for determining a three-dimensional displacement of claim 2, **characterized in that** the means for defining the second relative position of the castings (11, 14) comprise a device for digitizing the surfaces of the castings and image processing means.

5. The device for determining a three-dimensional displacement of any of claims 2 to 4, **characterized in that** the localization device is formed of an optical locator (17), the locatable units (12, 15, 21) including infrared-emitting diodes (23).

6. The device for determining a three-dimensional displacement of any of claims 2 to 4, **characterized in that** the localization device (17) is formed of several video cameras associated with image processing means, the locatable units being formed of graphic patterns.

7. A system for simulating of an orthognatic surgery action, **characterized in that** it includes:
an X-ray scanner;
a device of determination of a three-dimensional movement between relative first and second positions of two dental castings (11, 14), according to any of claims 2 to 6; and
an image processing system for combining tomographic images provided by the scanner with positioning data provided by the localization device (17) to obtain three-dimensional images.

8. The simulation system of claim 7, **characterized in that** the relation between the first and second referentials is determined from an additional unit (21) of a reference element (20) adapted to being mechanically associated to the guide mark (3) of the first interscupidation plate (1).

9. The simulation system of claim 7 or 8, **characterized in that** the guide mark (3) of the first interscupidation plate (1) is rigidly coupled with a third locatable unit which defines a second referential associated with the first plate (1).
